# FASCICULE DE BREVET EUROPEEN

(11) **EP 0 668 067 B1**
(45) Date de publication et mention de la délivrance du brevet: **09.09.1998**
(21) Numéro de dépôt: 95400368.7
(22) Date de dépôt: 22.02.1995
(51) Int. Cl.: A61F 13/15

(54) **Article d'hygiène féminine préplié**
Vorgefalteter Hygièneartikel für Frauen
Prefolded article for feminine hygiène

(30) Priorité: 22.02.1994 FR 9401964
(43) Date de publication de la demande: 23.08.1995
(73) Titulaire: FORT JAMES FRANCE, 68320 Kunheim (FR)
(72) Inventeur: Lefebvre du Grosriez, Carol, F-78600 Maisons Lafitte (FR)
(74) Mandataire: David, Daniel

(56) Documents cités:
- EP-A- 0 302 523
- WO-A-91/18574

## Description

L'invention concerne généralement un article d'hygiène féminine préplié.

Cet article est formé d'un corps central allongé comprenant une couche de surface perméable aux fluides, une couche imperméable aux fluides et une couche absorbante disposée entre les couches perméable et imperméable précitées, et le cas échéant, est muni d'au moins un volet s'étendant latéralement par rapport au corps central.

Les protections périodiques actuellement sur le marché, lorsqu'elles sont présentées emballées dans un sachet individuel, sont généralement pliées transversalement en trois ou en C.

Ces serviettes comportent des moyens adhésifs sous forme de bandes ou pavés d'adhésifs disposés sous la couche imperméable de la serviette, plus précisément du corps central, et destinés à fixer la serviette sur le sous-vêtement. Avant utilisation, ces moyens adhésifs sont protégés par exemple par une bande siliconée pelable. En ce qui concerne les serviettes munies de volets latéraux servant notamment à améliorer le maintien de la serviette sur le sous-vêtement, chaque volet comporte également un moyen adhésif et un moyen de protection de cet adhésif.

De ce fait, de nombreux matériaux et éléments distincts sont nécessaires pour la fabrication de ces serviettes. Leur utilisation entraîne plusieurs manipulations : ouvrir le sachet individuel, retirer la serviette du sachet, déplier la serviette et enfin retirer successivement la bande siliconée disposée sous le corps central et les autres petites bandes siliconées disposées sous chacun des volets latéraux alors que l'utilisatrice ne dispose que de deux mains. En outre, la fixation de la serviette par adhésifs, sur le sous-vêtement provoque l'usure et parfois la détérioration de ce dernier.

Par ailleurs, de manière générale, le pliage en trois ou en C ne confère pas à la serviette une fois dépliée une quelconque forme. Les serviettes qui sont, le cas échéant, tridimensionnelles, sont déjà formées ou conformées avant d'être emballées.

Le brevet européen B-0 344 229 révèle une serviette comprenant un corps central et deux volets latéraux qui avant et après utilisation de la serviette, sont repliés sur la serviette elle-même pliée en trois ou en C de manière à former un conditionnement et un sachet jetable. On élimine ainsi un élément : le sachet individuel indépendant. Cependant, cette serviette une fois dépliée ne prend pas une forme particulière dans l'espace, découlant de son mode de pliage et de son emballage dans les volets latéraux.

Les demandes de brevet internationales WO 91/18574 et WO 93/09743 décrivent une serviette périodique emballée dans un matériau siliconé formant à la fois un moyen de protection des adhésifs disposés sous la serviette et une pochette pour emballer la serviette, la serviette étant pliée transversalement en trois. Mais ce type de serviette est également plate et ne présente pas, une fois dépliée, de forme tridimensionnelle.

Ces différents produits bien que sous une forme pliée en trois et emballés dans un sachet, restent toutefois encombrants et peu discrets, en l'occurrence lorsqu'il s'agit de serviettes du type mince ou "ultra", qui gardent une fois pliée une surface non négligeable.

Par ailleurs, des serviettes prépliées prenant une forme tridimensionnelle ont été décrites notamment dans la demande de brevet français -A-2 463 610 et le brevet américain n° 4,067,336. Ces brevets ont plus précisément pour objet une protection périnéale formée d'une feuille souple de forme allongée, munie de plusieurs lignes de pliage prédéterminées suivant lesquelles la feuille est pliée avant usage. Ces lignes comprennent une ligne principale de pliage, centrale, dans la direction longitudinale de la feuille, une première paire de lignes de pliage divergeant vers l'arrière et ayant leur origine en un point commun situé sur la ligne principale, ces lignes allant jusqu'au pourtour de la feuille. Une seconde paire de lignes de pliage divergeant vers l'arrière est disposée entre la première paire de lignes divergentes et les bords latéraux de la feuille, et a son origine en un même point commun sur la ligne principale que la première paire de lignes de pliage. Les différents pliages sont réalisés de manière que la feuille formant protection prenne une forme anatomique.

Toutefois cet agencement de lignes de pliage et sa réalisation sont assez complexes, et ce type de serviette, plus précisément, protection périnéale, n'est pas prévue pour être emballée sous une forme pliée.

Un autre article absorbant jetable décrit dans la demande de brevet international WO 93/15700 consiste en deux corps souples et plats qui, sous une forme emballée, peuvent reposer l'un à côté de l'autre, les bords étant à plat. Ils sont prévus pour se placer contre le corps de l'utilisatrice sous une forme tridimensionnelle, lorsque l'article est mis en place. Les deux corps sont reliés l'un à l'autre par un bord commun qui forme une ligne de pliage autour de laquelle les corps sont pliés à l'état déballé et qui comprend un segment incurvé adoptant une forme convexe lorsque l'article est emballé en regardant dans une direction allant du bord commun vers un bord opposé d'un des deux corps.

Cette serviette prépliée prend une forme tridimensionnelle anatomique une fois retirée de l'emballage et dépliée pour être utilisée, mais elle n'est pas prépliée au cours de son conditionnement.

La serviette n'est pas non plus directement enveloppée dans un conditionnement ou sachet faisant partie intégrante de l'article. La serviette est emballée simplement en superposition à plat avec d'autres serviettes, par exemple dans un sac.

La présente invention a pour but de pallier l'ensemble des inconvénients précités en proposant un article d'hygiène féminine qui est préplié simultanément à son conditionnement et qui, une fois déballé et déplié adopte une forme tridimensionnelle.

L'invention permet ainsi d'éviter l'insertion de moyens de déformation, le moulage ou la conformation d'une serviette tridimensionnelle avant son conditionnement dans un emballage individuel.

L'invention a également pour but de fournir un article d'hygiène féminine emballé dans des moyens solidaires de ce même article, formant un emballage individuel.

L'invention a encore pour but d'éliminer les moyens de protection des adhésifs et le cas échéant les moyens adhésifs eux-mêmes, prévus classiquement sous le corps central de la serviette, ce qui permet d'éviter la détérioration ou l'usure des sous-vêtements par les adhésifs.

De plus, l'invention a pour but de réduire le nombre d'éléments composant la serviette, diminuant ainsi les coûts en matière première et facilitant considérablement la manipulation du produit lors de sa mise en place.

L'invention a en outre pour but de fournir un article d'hygiène féminine emballé individuellement de très faible encombrement, facile à transporter et très discret.

Enfin, l'invention a pour but de fournir un article préplié au moyen d'un procédé simple de pliage de l'article dans son emballage individuel.

Selon une caractéristique essentielle de l'invention, le corps central allongé de l'article d'hygiène féminine préplié comprend deux lignes de pliage transversales et une ligne de pliage longitudinale et centrale, divisée en trois segments délimités par lesdites lignes de pliage transversales, les parties du corps central le long de deux segments voisins étant destinées à être pliées l'une vers l'extérieur, l'autre, vers l'intérieur ou vice-versa.

On entend ici et dans la description qui suit, par l'expression "plié vers l'extérieur", le fait que, lorsque l'article est plié longitudinalement, la couche destinée à être mise sur le sous-vêtement se situe à l'intérieur du pli, et par l'expression "plié vers l'intérieur", le fait que, lorsque l'article est plié longidudinalement, la couche perméable côté corps, se situe à l'intérieur du pli.

Selon une autre caractéristique essentielle de l'invention, l'article est muni d'au moins un volet latéral.

Selon une caractéristique avantageuse de l'invention, l'article est emballé, avant utilisation, dans le volet latéral imperméable dont est muni l'article, au moyen d'un procédé consistant à plier transversalement, en trois ou en C, le corps central suivant les deux lignes de pliage transversales précitées, plier longitudinalement en deux ledit corps central plié en trois suivant les trois segments précités superposés de ladite ligne longitudinale et centrale de pliage, et envelopper ledit corps plié en quatre dans ledit volet latéral.

Selon une caractéristique préférentielle de l'invention, la partie du corps central le long du segment central de la ligne de pliage longitudinale et centrale précitée, est destinée à être pliée vers l'extérieur et les deux autres parties le long des segments disposés de part et d'autre dudit segment central, sont destinées à être pliées vers l'intérieur.

Selon une autre caractéristique avantageuse de l'invention, le corps central dudit article présente une partie centrale convexe, dirigée vers le haut, et deux parties avant et arrière disposées de part et d'autre de ladite partie centrale, concaves, dirigées vers le bas.

Selon encore une autre caractéristique préférentielle de l'invention, la partie du corps central le long du segment central de ladite ligne de pliage longitudinale et centrale est destinée à être pliée vers l'intérieur et les deux autres parties le long des segments disposés de part et d'autre dudit segment central sont destinées à être pliées vers l'extérieur.

Selon encore une caractéristique avantageuse de l'invention, le corps central dudit article présente une partie centrale concave, dirigée vers le bas, et deux parties avant et arrière de part et d'autre de ladite partie centrale, convexes, dirigées vers le haut.

Enfin, suivant une autre caractéristique de l'invention, seul le volet latéral comporte des moyens adhésifs.

D'autres caractéristiques et avantages de l'invention apparaîtront plus clairement à la lecture détaillée de l'invention qui suit et des figures dans lesquelles :
- la figure 1 représente un article d'hygiène féminine selon l'invention, à plat, qui n'est pas préplié, et muni d'un volet latéral unique,
- la figure 2 illustre le pliage en trois ou en C de l'article représenté à la figure 1,
- la figure 3 illustre le pliage longitudinal de l'article plié en trois représenté à la figure 2,
- la figure 4 illustre l'article des figures 1 à 3, emballé dans le volet latéral,
- la figure 5 représente toujours ce même article, préplié, à l'état déplié au porter, placé sur un sous-vêtement,
- la figure 6 représente un article selon l'invention suivant un autre mode de réalisation, à plat, sans prépliage et muni de deux volets latéraux disposés asymétriquement l'un par rapport à l'autre,
- la figure 7 illustre le pliage en trois de l'article représenté en figure 6,
- les figures 8 et 9 montrent l'emballage de l'article représenté en figure 7 dans les volets latéraux, après pliage longitudinal,
- la figure 10 illustre l'article préplié des figures 7 à 9, à l'état déplié, au porter, placé sur un sous-vêtement,
- la figure 11 représente encore un autre mode de réalisation de l'article selon l'invention, à plat, sans prépliage, et muni de deux volets latéraux, disposés symétriquement l'un par rapport à l'autre,
- la figure 12 illustre l'article représenté à la figure 11, plié en trois,
- la figure 13 montre le pliage des volets latéraux, une fois l'article des figures 11 et 12, plié en trois,
- la figure 14 représente l'article selon la figure 13, plié longitudinalement,
- les figures 15a et 15b représentent l'article préplié des figures 12 à 14 à l'état déplié en position sur un sous-vêtement, respectivement vue de dessus et vue de dessous, et
- la figure 16 représente un autre mode de réalisation de l'article selon l'invention, à plat, préplié, sans volets latéraux,
- la figure 17 représente l'article de la figure 16 plié transversalement en trois ou en C, et
- la figure 18 montre l'article plié en trois de la figure 17, plié longitudinalement.

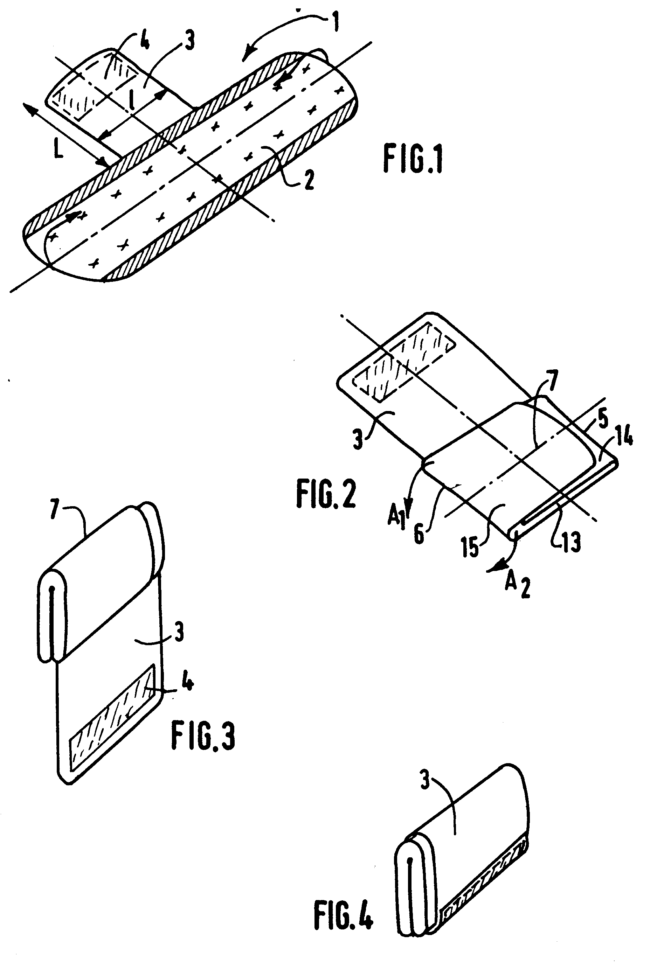

Suivant la figure 1, l'article d'hygiène féminine externe 1, représenté avant prépliage, comprend un corps central sensiblement allongé 2 muni d'un volet 3 s'étendant latéralement de la partie médiane du corps central 2. Ce volet latéral comporte un moyen de fixation 4 par exemple sous la forme d'un pavé d'adhésif. L'article représenté ici à plat n'est pas préplié.

Le corps central 2 a la structure d'une serviette périodique classique, c'est-à-dire comprend une couche de surface perméable aux fluides corporels destinée à être au contact du corps, une couche imperméable aux liquides corporels, destinée à être placée contre le sous-vêtement et une couche absorbante disposée entre les couches perméable et imperméable. Le volet latéral comprend au moins une couche imperméable et, le cas échéant, une couche perméable en surface.

De préférence, l'article est une serviette périodique mince, correspondant aux types de serviettes dénommées "ultra" dans le commerce. Les différents matériaux pouvant être utilisés pour fabriquer cet article seront décrits plus en détails dans la description qui suit.

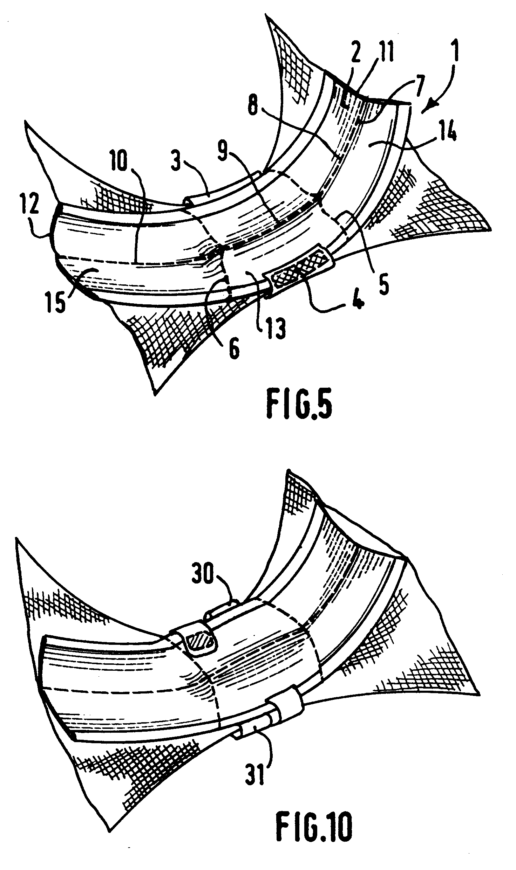

La figure 5 illustre l'article préplié, à l'état déplié, et en position au porter sur un sous-vêtement.

Les lignes de pliage apparaissent : d'une part, les lignes de pliage transversales 5 et 6 et d'autre part, la ligne de pliage longitudinale et centrale 7 s'étendant le long de l'axe central du corps central.

Cette ligne de pliage longitudinale se divise en trois segments 8, 9 et 10. Le premier segment 8 est délimité par l'extrémité 11 du corps central et la ligne de pliage transversale 5. Le second segment ou segment central 9 se situe entre les deux lignes de pliage transversales 5 et 6 et le troisième segment 10 se situe entre la ligne de pliage transversale 6 et l'autre extrémité 12 du corps central. Le volet latéral 3 unique est rabattu sous le sous-vêtement et est fixé par le moyen de fixation 4 sur la bordure latérale du corps central 2 située à l'opposé de la bordure latérale adjacente au volet latéral 3.

Une fois déplié, l'article représenté en figure 5 comprend une partie centrale et médiane 13 au niveau ou le long du segment central 9, qui est convexe, dirigée vers le haut (cette partie étant prépliée vers l'extérieur), et deux parties 14 et 15 au niveau ou le long, respectivement des segments 8 et 10, disposées de part et d'autre de la partie centrale 13, qui sont concaves, dirigées vers le bas (ces parties étant prépliées vers l'intérieur). L'article selon ce mode de réalisation, a donc une forme tridimensionnelle sensiblement anatomique en ayant une partie centrale et médiane protubérante venant au contact du corps dans la région urogénitale, se rapprochant de la source d'écoulement des fluides, et ayant des parties avant et arrière en forme de cuvette.

Cet article bien adapté au corps de la femme, se met et se maintient bien en place. Par conséquent, il est confortable et ne se déplace pas au porter. Cet article a également pour avantage de ne pas détériorer les sous-vêtements. En effet, aucun moyen de fixation tel qu'un adhésif n'est prévu pour fixer directement l'article au sous-vêtement. Seul le volet latéral 3 comporte un moyen de fixation 4 sur sa face arrière, qui sera fixé sur l'article lui-même.

Cette forme anatomique spécifique est engendrée directement par le pliage de l'article et son conditionnement dans un emballage individuel. En effet, le corps central est préformé au cours du pliage de l'article et adopte naturellement au dépliage une forme tridimensionnelle qu'il garde lorsqu'il est placé sur le sous-vêtement.

Les différentes étapes du procédé de pliage et emballage de l'article sont données ci-après suivant les figures 2 à 4.

Le corps central 2 de l'article est plié en trois ou en C suivant les lignes de pliage transversales 5 et 6, la partie 14 étant rabattue sur la partie centrale 13 suivant la ligne de pliage 5 et la partie 15 étant rabattue sur la partie 14 repliée, suivant la ligne de pliage transversale 6 ou vice-versa.

Ensuite, le corps central plié en trois, est plié longitudinalement en deux (suivant les flèches A1 et A2), vers l'extérieur, suivant la ligne de pliage longitudinale et centrale 7 et plus précisément suivant les trois segments 8, 9 et 10 superposés.

Au cours du pliage longitudinal du corps central, vers l'extérieur, le volet latéral 3 s'étend dans le plan de pliage longitudinal du corps central. De par ce pliage vers l'extérieur, la partie centrale 13 comportant le segment central 9 a une forme convexe, dirigée vers le haut, à l'état déplié, alors que les parties avant et arrière 14 et 15 comportant respectivement les segments 8 et 10 ont une forme concave, dirigée vers le bas.

Enfin, l'article est emballé, tel que représenté à la figure 4, dans le volet latéral 3. Ce dernier a une surface supérieure à la surface totale du corps central plié en quatre. Les dimensions de ce volet latéral sont telles que la longueur L du volet est supérieure à la largeur du corps central, et la largeur 1 du volet correspond sensiblement à la longueur de la partie centrale 13 de l'article, qui elle-même est de préférence égale au tiers de la longueur maximale du corps central. Le volet est enroulé autour de l'article ou corps central plié en quatre et forme une enveloppe protectrice. Le produit plié et emballé a une surface moitié moins grande qu'une serviette classiquement pliée en trois ou en C. L'article selon l'invention est ainsi peu encombrant, pratique à transporter et très discret.

Son utilisation est simplifiée car le nombre d'éléments à manipuler est moins important que celui des produits de l'art antérieur. Il suffit d'ouvrir l'emballage en désolidarisant le volet latéral enroulé du corps central plié, par exemple en décollant ce volet latéral, de déplier ensuite l'article, et de le positionner directement sur le sous-vêtement sans retrait de quelqu'élément que ce soit, tel que des moyens de protection des moyens de fixation. En position, le volet latéral est enroulé autour du sous-vêtement et vient se fixer sur la bordure latérale du corps central de l'article. Après utilisation, la fixation du volet sur la bordure du corps central est enlevée, l'article est retiré et peut être replié sur lui-même et emballé dans le volet latéral pour être jeté.

Un autre mode de réalisation de l'article, proche de celui qui vient d'être décrit, prévoit deux volets latéraux disposés symétriquement de part et d'autre de la partie médiane du corps central, l'un des volets étant de longueur plus courte que l'autre. Un seul des volets comporte un moyen de fixation permettant de fixer les deux volets l'un à l'autre, une fois qu'ils sont rabattus sous le sous-vêtement lorsque l'article est mis en place.

Selon encore un autre mode de réalisation de l'article selon l'invention, ce dernier comprend un corps central 20 et deux volets latéraux 30 et 31 disposés latéralement et asymétriquement de part et d'autre de la partie médiane du corps central 20. Chacun des volets latéraux 30 et 31 comporte sur sa face inférieure un moyen de fixation, respectivement 40 et 41, destiné à venir se fixer sur chacune des bordures latérales du corps central 20 en vis à vis du côté opposé à chacune des bordures latérales du corps, adjacente au volet correspondant.

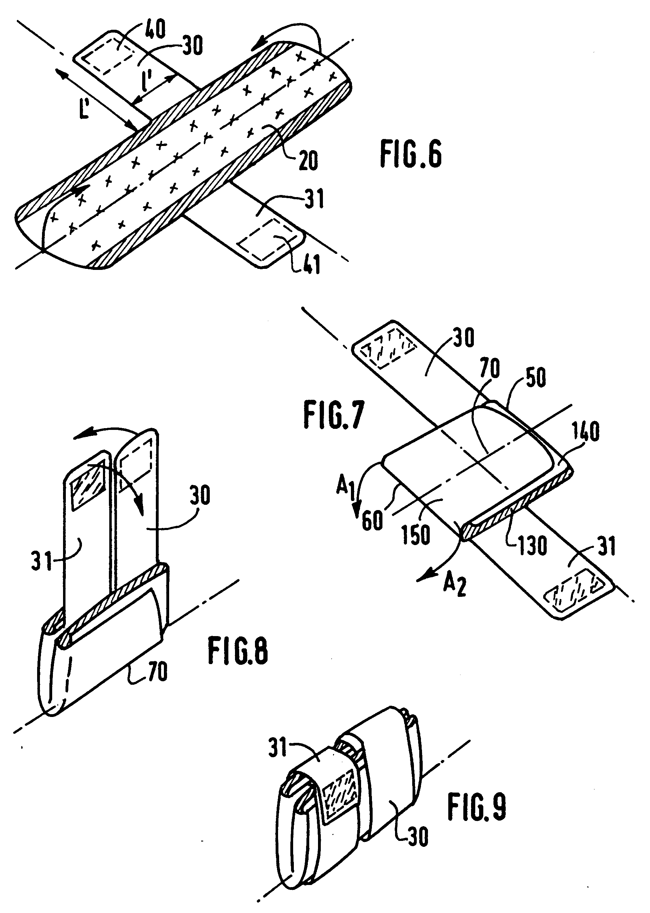

La figure 7 illustre le pliage en trois de cet article suivant les lignes de pliage transversales 50 et 60, la partie 140 du corps central étant rabattue sur la partie centrale 130 du corps suivant la ligne de pliage 50 et la partie 150 étant rabattue sur la partie 140 déjà rabattue, suivant la ligne de pliage 60. La figure 8 illustre l'article plié longitudinalement, suivant la ligne de pliage longitudinale 70. Les volets latéraux 30 et 31 s'étendent dans le plan de pliage longitudinal. La surface totale des volets est prévue de manière à recouvrir totalement la surface de l'article plié en quatre. Les dimensions d'un volet latéral sont telles que la longueur L' est supérieure à la largeur du corps central et la largeur l' d'un volet correspond sensiblement à la moitié de la longueur de la partie centrale 130 du corps central, qui elle-même est de préférence égale au tiers de la longueur maximale du corps central. Enfin, l'article plié en quatre, est également emballé ici dans les deux volets latéraux 30 et 31 comme représenté en figure 9. Les moyens de fixation prévus sur chacun des volets permettent de fixer les volets enroulés autour de l'article.

La figure 10 représente l'article déplié au porter, en position sur un sous-vêtement. La partie centrale et médiane 130 du corps central a une forme convexe, dirigée vers le haut, alors que les parties 140 et 150 situées de part et d'autre de la partie centrale 130 ont des formes concaves, dirigées vers le bas.

L'invention comprend également un autre mode de prépliage qui procure une autre forme à l'article selon l'invention.

En résumé, ce mode de réalisation diffère du précédent par le simple fait que le corps central de l'article plié en trois, est plié longitudinalement en deux vers l'intérieur, et non pas vers l'extérieur.

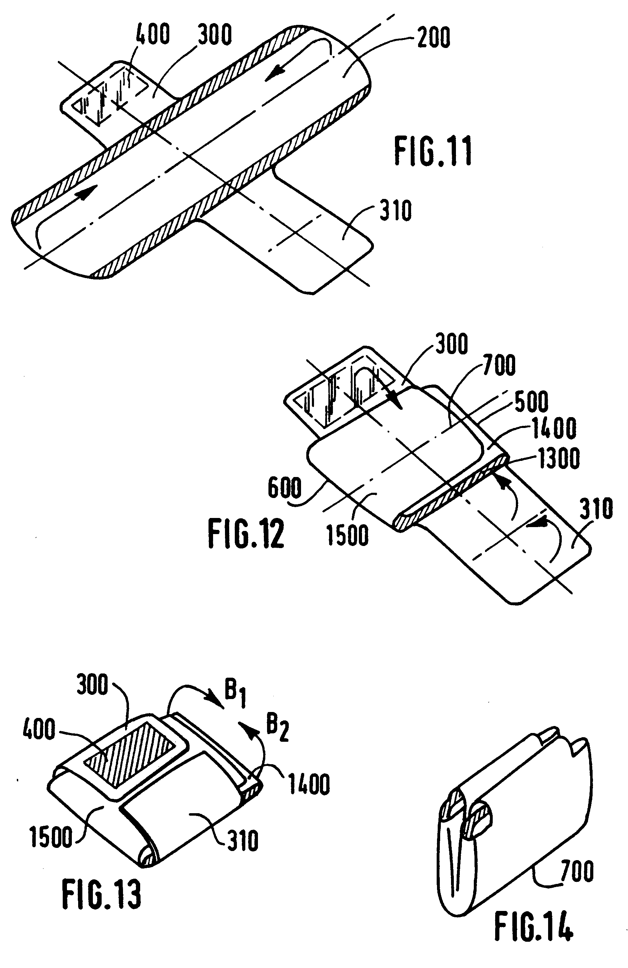

La figure 11 illustre cet article à plat sans prépliage. Il comprend un corps central 200 muni de deux volets latéraux 300 et 310 de longueur différente et disposés symétriquement de part et d'autre de la partie médiane du corps central.

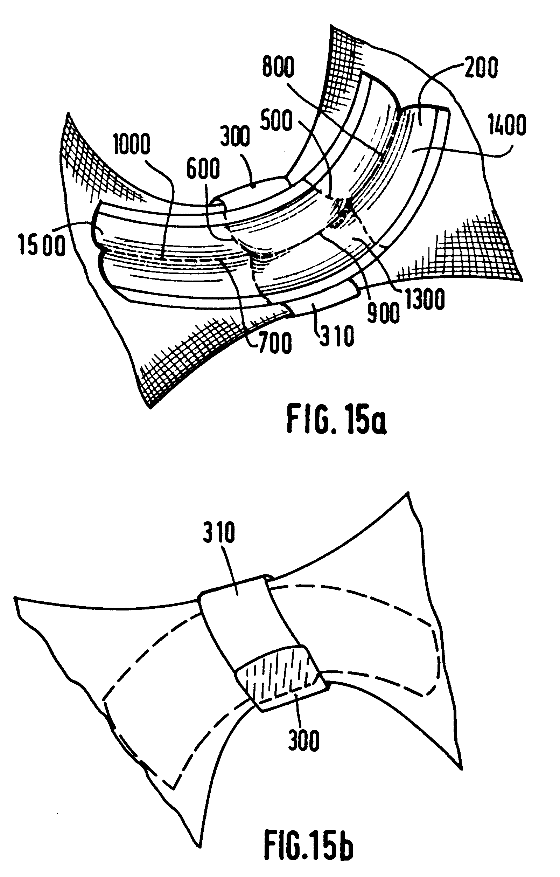

Ce même article préplié à l'état déplié et en place sur un sous-vêtement est représenté à la figure 15a. Il comprend deux lignes de pliage transversales 500 et 600 et une ligne de pliage longitudinale et centrale 700. Cette dernière est également divisée en trois segments 800, 900 et 1000 délimités par les lignes de pliage transversales 500 et 600.

La partie centrale et médiane 1300 comportant le segment 900, a une forme concave, dirigée vers le bas, alors que les parties avant et arrière 1400 et 1500 sont de forme convexes, dirigées vers le haut. Cet article est également de forme trimensionnelle mais adopte une section en cuvette dans sa partie médiane. Ce genre de serviette périodique constitue un réceptable et améliore la rétension des fluides corporels.

La figure 15b montre la fixation des deux volets latéraux entre eux sous le sous-vêtement, sans adhérence directe à ce dernier.

Le prépliage particulier de cet article est obtenu au moyen du procédé de pliage qui suit.

En référence à la figure 12, le corps central est d'abord plié en trois suivant les lignes de pliage transversales 500 et 600, la partie 1400 du corps central étant rabattue sur la partie centrale 1300 suivant la ligne de pliage 500 puis la partie 1500 étant rabattue sur la partie 1400 repliée du corps, suivant la ligne de pliage transversale 600.

Suivant une étape intermédiaire, le volet 310 de plus grande longueur est replié sur lui-même en deux puis replié sur la moitié de la partie 1500 du corps central plié en trois, l'autre volet latéral 300 est replié sur l'autre moitié de la partie 1500. Puis le corps central déjà plié en trois est plié longitudinalement en deux (suivant les flèches B1 et B2), vers l'intérieur suivant la ligne de pliage longitudinale 700 comme l'illustrent les figures 13 et 14 ou encore suivant les trois segments superposés 800, 900 et 1000. Dans ce mode de réalisation, l'article et plus précisément le corps central plié en quatre est complètement refermé par les volets latéraux, du fait du moyen de fixation 400 qui vient se placer sur l'autre volet latéral 310 préplié.

Dans ce mode de réalisation, on pourrait ne replier à l'étape intermédiaire que le volet latéral le plus court et enrouler le corps plié en quatre dans le volet latéral le plus long, en prévoyant le moyen de fixation uniquement sur ce dernier pour fixer le volet une fois enroulé sur le corps central plié et former un conditionnement.

Dans le cas de figure où le ou les volets latéraux ne seraient pas repliés avant l'étape de pliage longitudinal, sur le corps central déjà plié en trois, le ou les volets au cours du pliage longitudinal, iraient s'étendre respectivement dans les plans formés par les faces externes correspondantes de la partie centrale du corps central plié longitudinalement.

Des modes de réalisation proches de celui qui vient d'être décrit englobent des articles dont le corps central est muni d'un volet latéral unique ou encore de deux volets latéraux disposés asymétriquement l'un par rapport à l'autre de part et d'autre de la partie médiane du corps central. Le volet unique ou les deux volets asymétriques peuvent être également enroulés autour du corps central plié en quatre pour former un emballage protecteur.

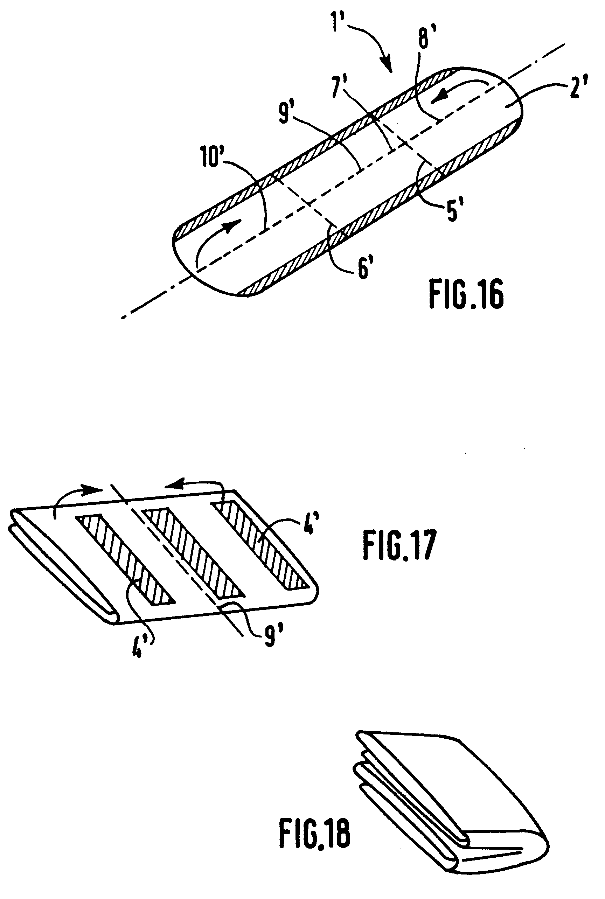

Enfin, un dernier mode de réalisation de l'article selon l'invention est illustré par les figures 16 à 18, qui représentent un article d'hygiène féminine ne comportant pas de volets latéraux.

Cet article d'hygiène féminine 1' comme le montre la figure 16, comprend un corps central allongé 2' comportant deux lignes de pliage transversales 5' et 6' et une ligne de pliage longitudinale et centrale 7' elle-même comprenant trois segments 8', 9' et 10' délimités par les lignes de pliage transversales 5' et 6'.

Le corps central 2' est plié en trois ou en C suivant les lignes de pliage transversales 5' et 6' comme les articles des modes de réalisation précédemment décrits. Puis l'article plié en trois est à son tour plié longitudinalement en deux, soit vers l'intérieur, soit vers l'extérieur (voir la figure 18), suivant les segments 8', 9' et 10' superposés de la ligne de pliage longitudinale et centrale 7'.

Par contre ici, des moyens de fixation 4' prévus au dos de l'article dans sa partie médiane, sont nécessaires pour, d'une part, tenir en position pliée et fermée l'article plié en quatre et, d'autre part, fixer l'article sur un sous-vêtement, en cours d'utilisation.

Cet article a une forme tridimensionnelle et, une fois déplié, présente suivant le pliage longitudinal effectué, une partie centrale et médiane soit protubérante, soit en forme de cuvette. Cependant, cet article ne sera pas totalement protégé par un emballage.

Les matériaux utilisés pour la fabrication d'une telle serviette sont par exemple les matériaux classiquement utilisés dans la structure des serviettes hyperminces ou "ultra".

La couche de surface est en matériau perméable aux fluides corporels et suffisamment doux pour être au contact de la peau, tel qu'un film ou un voile perforé ou encore un nontissé perméable aux fluides.

La couche imperméable située à l'arrière de l'article d'hygiène est constituée d'un matériau imperméable aux liquides corporels, tel qu'une feuille de polyéthylène éventuellement gaufrée. Elle peut également être en un matériau de type complexe formé d'un nontissé constitué de fibres liées, associé à un autre nontissé de type "meltblown" composé de microfibres. Tout matériau ayant la propriété d'être imperméable aux liquides corporels et perméable à la vapeur d'eau est approprié.

La couche absorbante ou matelas absorbant est quant à lui sous une forme allongée, de préférence rectangulaire et est constituée d'un matériau cellulosique incluant des composants super-absorbants. Par exemple, le matelas absorbant est formé d'une bande de fibres cellulosiques, papetières, obtenue par voie sèche et liée par un latex, c'est-à-dire un matériau de type "air-laid", dont les deux bords latéraux sont rabattus sur une partie centrale sur laquelle est déposé au préalable le matériau super-absorbant. Ce dernier peut être sous la forme de poudre, de fibres, de feuille et également sous la forme de poudre liée à des fibres synthétiques.

D'autres matériaux tels qu'une mousse synthétique ou naturelle, par exemple de sphaigne traitée, présentant de grandes capacités d'absorption, peuvent être utilisés. Le traitement de la sphaigne mentionnée ci-dessus est décrit dans la demande de brevet européen n° 0 546 585.

La couche de surface et la couche imperméable sont associées par exemple par scellage pour former le matelas.

De manière générale, les matériaux constituant le ou les volets latéraux doivent être suffisamment souples ou flexibles.

Ces volets latéraux sont au moins constitués d'une couche imperméable ou peu perméable permettant de protéger l'article, lorsqu'ils sont enroulés autour du corps central pour former une enveloppe ou un emballage de l'article.

Ils peuvent également, le cas échéant, comporter en surface une feuille relativement perméable, par exemple de nontissé, dans le mode de réalisation représenté aux figures 13 et 14 où les volets latéraux sont rabattus sur le corps central plié en trois avant l'étape de pliage longitudinal.

Enfin, ces volets peuvent être de type rapporté c'est-à-dire fixés sur la face du corps central destinée à être en contact avec le sous-vêtement. Ils sont de préférence de type intégré, c'est-à-dire, inclus dans la structure du corps central, en étant formés par exemple d'une extension latérale de la couche imperméable du corps central.

Les moyens de fixation prévus sur les volets latéraux peuvent être des adhésifs déposés sous forme de pavés, de préférence auto-adhésifs sur le matériau constituant les volets latéraux pour un retrait facile lorsque l'on ouvre l'emballage pour déballer l'article et lorsque l'on retire l'article en position sur le sous-vêtement, après usage.

Dans les modes de réalisation représentés aux figures 5 et 10, les moyens de fixation sous forme d'adhésifs doivent également être autoadhésifs sur le matériau constituant les bordures latérales du corps central. Ces bordures peuvent être constituées par exemple d'un retour de la couche imperméable située à l'arrière du corps central.

Ces moyens de fixation peuvent également être constitués d'un dispositif commercialisé sous la marque VELCRO.

Enfin, il est à noter que les articles selon l'invention ne comportent pas, avant utilisation, de moyens de protection des moyens de fixation, tel que des bandes siliconées.

## Revendications

1. Article d'hygiène féminine (1, 1') préplié, formé d'un corps central allongé (2, 2', 200), comprenant une couche perméable aux fluides en surface, une couche imperméable aux fluides et une couche absorbante disposée entre les couches perméable et imperméable précitées, caractérisé en ce que ledit corps central (2, 2', 200) comprend deux lignes de pliage transversales (5, 5', 500) et (6, 6', 600) et une ligne de pliage longitudinale et centrale (7, 7', 700), divisée en trois segments (8, 8', 800), (9, 9', 900) et (10, 10', 1000) délimités par lesdites lignes de pliage transversales (5, 5', 500) et (6, 6', 600), les parties du corps central le long de deux segments voisins étant destinées à être pliées l'une vers l'extérieur, l'autre, vers l'intérieur, ou vice-versa.

2. Article selon la revendication 1, caractérisé en ce qu'il est muni d'au moins un volet latéral (3, 300).

3. Article selon la revendication 1, caractérisé en ce qu'avant utilisation, l'article est préplié au moyen d'un procédé consistant à :
- plier en trois ou en C le corps central (2') suivant les deux lignes de pliage transversales précitées (5') et (6'), et
- plier longitudinalement en deux ledit corps (2') plié en trois, suivant les trois segments précités (8'), (9') et (10') superposés de la ligne longitudinale et centrale (7') de pliage.

4. Article selon la revendication 2, caractérisé en ce qu'avant utilisation, l'article est emballé dans ledit volet latéral (3) qui est imperméable, au moyen d'un procédé consistant à :
- plier transversalement en trois ou en C le corps central (2) suivant les deux lignes de pliage transversales précitées (5) et (6).
- plier longitudinalement en deux ledit corps (2) plié en trois, suivant les trois segments précités (8), (9) et (10) superposés de la ligne longitudinale et centrale (7) de pliage, et
- envelopper ledit corps plié en quatre dans ledit volet latéral (3).

5. Article selon la revendication 2, caractérisé en ce qu'avant utilisation, l'article est préplié au moyen d'un procédé consistant à :
- plier en trois ou en C le corps central (200) suivant les deux lignes de pliage transversales précitées (500) et (600),
- plier le volet latéral (300, 310) sur ledit corps central (200) plié en trois, et
- plier longitudinalement en deux ledit corps central (200) plié en trois suivant les trois segments précités (800, 900, 1000) superposés de la ligne centrale longitudinale (700) de pliage.

6. Article selon l'une des revendications 2 et 4 à 5, caractérisé en ce que seul le volet latéral (3, 300) comporte des moyens de fixation (4, 400).

7. Article selon la revendication 1 ou 2, caractérisé en ce que la partie (13) du corps central (2) le long du segment central (9) de la ligne de pliage longitudinale centrale précitée (7), est destinée à être pliée vers l'extérieur et les deux autres parties (14) et (15) le long, respectivement des segments (8) et (10) disposés de part et d'autre dudit segment central (9), sont destinées à être pliées vers l'intérieur.

8. Article selon la revendication 4, caractérisé en ce que le corps central (2) plié en trois est plié longitudinalement en deux vers l'extérieur de manière que le volet latéral s'étende dans le plan de pliage longitudinal dudit corps central (2).

9. Article selon la revendication 7 ou 8, caractérisé en ce qu'à l'état déplié, le corps central (2) dudit article présente une partie centrale (13) convexe, dirigée vers le haut, et deux parties avant et arrière (14) et (15) disposées de part et d'autre de ladite partie centrale (13), concaves, dirigées vers le bas.

10. Article selon la revendication 1 ou 2, caractérisé en ce que la partie (1300) du corps central (200) le long du segment central (900) de ladite ligne de pliage longitudinale et centrale (700), est destinée à être pliée vers l'intérieur et les deux autres parties (1400) et (1500) le long, respectivement des segments (800) et (1000) disposés de part et d'autre dudit segment central (900), sont destinées à être pliées vers l'extérieur.

11. Article selon la revendication 4, caractérisé en ce que le corps central plié en trois est plié longitudinalement en deux vers l'intérieur de manière que le volet latéral s'étende dans le plan formé par la face externe de la partie correspondante pliée en deux dudit corps central.

12. Article selon la revendication 5, caractérisé en ce que le corps central (200) plié en trois et comportant au moins un volet latéral (300) replié sur ledit corps central (200) plié en trois, est plié longitudinalement vers l'intérieur.

13. Article selon l'une des revendications 10 à 12, caractérisé en ce qu'à l'état déplié, le corps central (200) dudit article présente une partie centrale (1300) concave, dirigée vers le bas, et deux parties avant et arrière (1400) et (1500) disposées de part et d'autre de ladite partie centrale (1300), convexes, dirigées vers le haut.

14. Article selon l'une des revendications 2 et 4 à 13, caractérisé en ce que ledit article est muni d'un volet latéral unique (3).

15. Article selon l'une des revendications 2 et 4 à 13, caractérisé en ce que ledit article est muni de deux volets latéraux (30, 300) et (31, 310).

16. Article selon la revendication 15, caractérisé en ce que les volets latéraux (300, 310) sont disposés symétriquement de part et d'autre du corps central (200) et sont de longueur différente.

17. Article selon la revendication 16, caractérisé en ce que le volet latéral (300) de plus petite longueur comporte un moyen de fixation (400) sur sa face arrière.

18. Article selon la revendication 16, caractérisé en ce que le volet latéral de plus grande longueur comporte un moyen de fixation sur sa face arrière.

19. Article selon la revendication 15, caractérisé en ce que les volets (30, 31) sont disposés de part et d'autre du corps central (20) de manière asymétrique et sont de même longueur.

20. Article selon la revendication 19, caractérisé en ce que chaque volet latéral (30, 31) comporte un moyen de fixation sur sa face arrière.

21. Article selon l'une des revendications 2, 4 à 20, caractérisé en ce que le ou les volets latéraux ont une surface totale supérieure à la surface totale externe du corps central plié en quatre.

## Claims

1. Prefolded article of feminine hygiene (1, 1'), formed of an elongated central body (2, 2', 200), comprising a layer permeable to fluids on the surface, a layer impermeable to fluids and an absorbent layer disposed between the aforementioned permeable and impermeable layers, characterized in that the said central body (2, 2', 200) includes two transverse fold lines (5, 5', 500) and 6, 6', 600) and a central longitudinal fold line (7, 7', 700) divided into three segments (8, 8', 800), (9, 9', 900) and (10, 10', 1000) delimited by the said transverse fold lines (5, 5', 500) and 6, 6', 600), the parts of the central body along two adjacent segments being intended to be folded, one outwards and the other inwards, or vice versa.

2. Article according to claim 1, characterized in that it is provided with at least one side flap (3, 300).

3. Article according to claim 1, characterized in that before use, the article is prefolded by means of a process consisting of :
- folding the central body (2') in three or in a C following the two aforementioned transverse fold lines (5') and (6'), and
- folding longitudinally in two the said body (2') folded in three, following the three aforementioned superimposed segments (8'), (9') and (10') of the central longitudinal fold line (7').

4. Article according to claim 2, characterized in that before use, the article is wrapped in the said side flap (3) which is impermeable, by means of a process consisting of :
- folding the central body (2) transversely in three or in a C following the two aforementioned transverse fold lines (5) and (6),
- folding longitudinally in two the said body (2) folded in three, following the three aforementioned superimposed segments (8), (9), (10) of the central longitudinal fold line (7), and
- wrapping the said body folded in four in the said side flap (3).

5. Article according to claim 2, characterized in that before use, the article is prefolded by means of a process consisting of :
- folding the central body (200) in three or in a C following the two aforementioned transverse fold lines (500) and (600),
- folding the side flap (300, 310) onto the said central body (200) folded in three, and
- folding longitudinally in two the said central body (200) folded in three, following the three aforementioned superimposed segments (800, 900, 1000) of the central longitudinal fold line (700).

6. Article according to one of claims 2 and 4 to 5, characterized in that only the side flap (3, 300) has means of attachment (4, 400).

7. Article according to claim 1 or 2, characterized in that the part (13) of the central body (2) along the central segment (9) of the aforementioned central longitudinal fold line (7) is intended to be folded outwards and the two other parts (14) and (15) along the segments (8) and (10) respectively disposed either side of the said central segment (9), are intended to be folded inwards.

8. Article according to claim 4, characterized in that the central body (2) folded in three is folded longitudinally in two outwards so that the side flap extends in the longitudinal fold plane of the said central body (2).

9. Article according to claim 7 or 8, characterized in that in the unfolded state, the central body (2) of the said article has a convex central part (13), directed upwards, and two concave front and rear parts (14) and (15) disposed either side of the said central part (13), directed downwards.

10. Article according to claim 1 or 2, characterized in that the part (1300) of the central body (200) along the central segment (900) of the said central longitudinal fold line (700), is intended to be folded inwards and the two other parts (1400) and (1500) along the segments (800) and (1000) respectively disposed either side of the said central segment (900), are intended to be folded outwards.

11. Article according to claim 4, characterized in that the central body folded in three is folded longitudinally in two inwards so that the side flap extends in a plane formed by the outer face of the corresponding part folded in two of the said central body.

12. Article according to claim 5, characterized in that the central body (200) folded in three and having at least one side flap (300) folded onto the said central body (200) folded in three, is folded longitudinally inwards.

13. Article according to one of claims 10 to 12, characterized in that in the unfolded state, the central body (200) of the said article has a concave central part (1300), directed downwards, and two front and rear convex parts (1400) and (1500) disposed either side of the said central part (1300), directed upwards.

14. Article according to one of claims 2 and 4 to 13, characterized in that the said article is provided with a single side flap (3).

15. Article according to one of claims 2 and 4 to 13, characterized in that the said article is provided with two side flaps (30, 300) and 31, 310).

16. Article according to claim 15, characterized in that the side flaps (300, 310) are disposed symmetrically either side of the central body (200) and are of different lengths.

17. Article according to claim 16, characterized in that the shorter side flap (300) has a means of attachment (400) on its rear face.

18. Article according to claim 16, characterized in that the longer side flap has a means of attachment on its rear face.

19. Article according to claim 15, characterized in that the flaps (30, 31) are disposed either side of the central body (20) in an asymmetrical manner and are of the same length.

20. Article according to claim 19, characterized in that each side flap (30, 31) has a means of attachment on its rear face.

21. Article according to one of claims 2 and 4 to 20, characterized in that the side flaps have a total surface area greater than the total external surface area of the central body folded in four.

## Patentansprüche

1. Vorgefalteter Artikel für weibliche Hygiene (1, 1'), gebildet von einem zentralen, länglichen Körper (2, 2', 200), der eine für Flüssigkeiten durchlässige Schicht an der Oberseite, eine für Flüssigkeiten undurchlässige Schicht und eine zwischen den vorgenannten durchlässigen und undurchlässigen Schichten angeordnete absorbierende Schicht aufweist, dadurch gekennzeichnet, daß der zentrale Körper (2, 2', 200) zwei transversale Faltlinien (5, 5', 500) und (6, 6', 600) und eine längsgerichtete und zentrale Faltlinie (7, 7', 700) aufweist, die in drei Segmente (8, 8', 800), (9, 9', 900) und (10, 10', 1000) unterteilt ist, die von den transversalen Faltlinien (5, 5', 500) und (6, 6', 600) begrenzt sind, wobei die Teile des zentralen Körpers längs zweier benachbarter Segmente dazu bestimmt sind, zum einen nach außen, zum anderen nach innen oder umgekehrt gefaltet zu werden.

2. Artikel gemäß Anspruch 1, dadurch gekennzeichnet, daß er mit zumindest einem seitlichen Flügel (3, 300) versehen ist.

3. Artikel gemäß Anspruch 1, dadurch gekennzeichnet, daß der Artikel vor Gebrauch vorgefaltet ist, mittels eines Verfahrens, bestehend aus:
- Falten in drei Abschnitte oder in Form eines C des zentralen Körpers (2') entlang der beiden vorerwähnten quergerichteten Faltlinien (5') und (6') und
- Falten in Längsrichtung in zwei Abschnitte des in drei Abschnitte gefalteten Körpers (2') entlang der drei vorerwähnten Segmente (8'), (9') und (10'), die auf der längsgerichteten und zentralen Faltlinie (7') übereinandergelagert sind.

4. Artikel gemäß Anspruch 2, dadurch gekennzeichnet, daß der Artikel vor dem Gebrauch in dem seitlichen Flügel (3) verpackt ist, der undurchlässig ist, mittels eines Verfahrens, bestehend aus:
- Falten in Querrichtung in drei Abschnitte oder in Form eines C des zentralen Körpers (2) entlang der beiden vorerwähnten, quergerichteten Faltlinien (5) und (6),
- Falten in Längsrichtung in zwei Abschnitte des in drei Abschnitte gefalteten Körpers (2) entlang der drei vorgenannten Segmente (8), (9) und (10), die auf der längsgerichteten und zentralen Faltlinie (7) übereinandergelagert sind, und
- Einhüllen des in vier Abschnitte gefalteten Körpers in den seitlichen Flügel (3).

5. Artikel gemäß Anspruch 2, dadurch gekennzeichnet, daß der Artikel vor Gebrauch vorgefaltet ist, mittels eines Verfahrens, bestehend aus:
- Falten in drei Abschnitte oder in Form eines C des zentralen Körpers (200) entlang der beiden vorgenannten, transversalen Faltlinien (500) und (600),
- Falten des seitlichen Flügels (300, 310) auf den in drei Abschnitte gefalteten zentralen Körper (200), und
- Falten in Längsrichtung in zwei Abschnitte des in drei Abschnitte gefalteten zentralen Körpers (200) entlang der drei vorgenannten Segmente (800, 900, 1000), die auf der zentralen, längsgerichteten Faltlinie (700) übereinandergelagert sind.

6. Artikel gemäß einem der Ansprüche 2 und 4 bis 5, dadurch gekennzeichnet, daß allein der seitliche Flügel (3, 300) Befestigungsmittel (4, 400) aufweist.

7. Artikel gemäß Anspruch 1 oder 2, dadurch gekennzeichnet, daß der Teil (13) des zentralen Körpers (2) entlang des zentralen Segmentes (9) der vorgenannten, längsgerichteten, zentralen Faltlinie (7) dazu bestimmt ist, nach außen gefaltet zu werden, und die beiden anderen Teile (14) und (15) jeweils längs der Segmente (8) und (10), die auf der einen und der anderen Seite des zentralen Segments (9) angeordnet sind, dazu bestimmt sind, nach innen gefaltet zu werden.

8. Artikel gemäß Anspruch 4, dadurch gekennzeichnet, daß der in drei Abschnitte gefaltete, zentrale Körper (2) in Längsrichtung in zwei Abschnitte so nach außen gefaltet ist, daß der seitliche Flügel sich in der Ebene der Faltung längs des zentralen Körpers (2) erstreckt.

9. Artikel gemäß Anspruch 7 oder 8, dadurch gekennzeichnet, daß im entfalteten Zustand der zentrale Körper (2) des Artikels einen zentralen konvexen Teil (13) aufweist, der nach oben gerichtet ist, und zwei hintere und vordere Teile (14) und (15), die beiderseits des zentralen Teils (13) angeordnet, konkav und nach unten gerichtet sind.

10. Artikel gemaß Anspruch 1 oder 2, dadurch gekennzeichnet, daß der Teil (1300) des zentralen Körpers (200) entlang des zentralen Segmentes (900) der längsgerichteten und zentralen Faltlinie (700) bestimmt ist, nach innen gefaltet zu werden, und die beiden anderen Teile (1400 und 1500) jeweils längs der Segmente (800) und (1000), die beiderseits des zentralen Segmentes (900) angeordnet sind, dazu bestimmt sind, nach außen gefaltet zu werden.

11. Artikel gemäß Anspruch 4, dadurch gekennzeichnet, daß der in drei Abschnitte gefaltete zentrale Körper in Längsrichtung in zwei Abschnitte nach innen gefaltet ist, so daß sich der seitliche Flügel in der Ebene erstreckt, die von der äußeren Seite des korrespondierenden, in zwei Abschnitte gefalteten Teils des Zentralkörpers gebildet ist.

12. Artikel gemaß Anspruch 5, dadurch gekennzeichnet, daß der zentrale Körper (200), der in drei Abschnitte gefaltet ist und zumindest einen seitlichen Flügel (300) aufweist, der auf den in drei Abschnitte gefalteten, zentralen Körper (200) gefaltet ist, in Längsrichtung nach innen gefaltet ist.

13. Artikel gemäß einem der Ansprüche 10 bis 12, dadurch gekennzeichnet, daß der zentrale Körper (200) des Artikels im entfalteten Zustand einen zentralen konkaven Teil (1300) zeigt, der nach unten gerichtet ist, und zwei vordere und hintere Teile (1400) und (1500), die beiderseits des zentralen, konvexen und nach unten gerichteten Teils (1300) angeordnet sind.

14. Artikel gemaß einem der Ansprüche 2 und 4 bis 13, dadurch gekennzeichnet, daß der Artikel mit einem einzigen seitlichen Flügel (3) versehen ist.

15. Artikel gemäß einem der Ansprüche 2 und 4 bis 13, dadurch gekennzeichnet, daß der Artikel mit zwei seitlichen Flügeln (30, 300) und (31, 310) versehen ist.

16. Artikel gemäß Anspruch 15, dadurch gekennzeichnet, daß die seitlichen Flügel (300, 310) symmetrisch auf der einen und der anderen Seite des zentralen Körpers (200) angeordnet sind und verschiedene Länge haben.

17. Artikel gemäß Anspruch 16, dadurch gekennzeichnet, daß der seitliche Flügel (300) von geringerer Länge ein Befestigungsmittel (400) auf seiner Rückseite aufweist.

18. Artikel gemäß Anspruch 16, dadurch gekennzeichnet, daß der seitliche Flügel von größerer Länge ein Mittel zur Befestigung auf seiner Rückseite aufweist.

19. Artikel gemäß Anspruch 15, dadurch gekennzeichnet, daß die Flügel (30, 31) auf beiderseits des zentralen Körpers (20) auf asymmetrische Weise angeordnet sind und dieselbe Länge aufweisen.

20. Artikel gemäß Anspruch 19, dadurch gekennzeichnet, daß jeder seitliche Flügel (30, 31) ein Mittel zur Befestigung auf seiner Rückseite aufweist.

21. Artikel gemäß einem der Ansprüche 2, 4 bis 20, dadurch gekennzeichnet, daß der oder die seitlichen Flügel eine gesamte Oberfläche haben, die größer als die gesamte äußere Oberfläche des in vier Abschnitte gefalteten zentralen Körpers ist.
